(19)

Europäisches
Patentamt

European
Patent Office

Office européen
des brevets

(11)     **EP 2 554 110 B1**

(12)     **EUROPEAN PATENT SPECIFICATION**

(45) Date of publication and mention
of the grant of the patent:
**30.07.2014  Bulletin 2014/31**

(21) Application number: **12188050.4**

(22) Date of filing: **21.01.2011**

(51) Int Cl.:
*A61B 5/00* (2006.01)        *A61B 5/145* (2006.01)
*A61B 5/1486* (2006.01)       *A61M 5/142* (2006.01)
*G06F 19/00* (2011.01)

(54) **Analyte testing method and system**

Analytentestverfahren und -system

Procédé et système d'essais d'analytes

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.01.2010  US 297573 P**

(43) Date of publication of application:
**06.02.2013  Bulletin 2013/06**

(62) Document number(s) of the earlier application(s) in
accordance with Art. 76 EPC:
**11702752.4 / 2 525 710**

(73) Proprietor: **Lifescan, Inc.
Milpitas, CA 95035 (US)**

(72) Inventors:
• **Ray, Pinaki
Fremont, CA 94539 (US)**
• **Matian, Greg
Foster City, CA 94404 (US)**
• **Price, David
Carlsbad CA 92009 (US)**
• **Rho, Hee, Jun
West Chester, PA 19380 (US)**
• **Harmon, Kirk
San Ramon, CA 94582 (US)**

(74) Representative: **Carpmaels & Ransford LLP
One Southampton Row
London WC1B 5HA (GB)**

(56) References cited:
WO-A1-2006/001929      WO-A1-2009/137661
US-A1- 2008 200 897     US-A1- 2008 235 053
US-B1- 6 554 798

## Description

## Background

Glucose monitoring is a fact of everyday life for people with diabetes. The accuracy of such monitoring can significantly affect the health and ultimately the quality of life for people with diabetes. Generally, a person with diabetes should measure blood glucose levels several times a day to monitor and control blood sugar levels. Failure to test blood glucose levels accurately and on a regular basis can result in serious diabetes-related complications, including cardiovascular disease, kidney disease, nerve damage and blindness. There are a number of electronic devices currently available which enable an individual to test the glucose level in a small sample of blood. One such glucose meter is the OneTouch® Ultra® glucose meter, a product which is manufactured by LifeScan.

In addition to glucose monitoring, people with diabetes often have to perform drug therapy such as, for example, insulin dosing. People with diabetes may self-administer insulin to reduce their blood glucose concentration. There are a number of mechanical devices currently available which enable an individual to dose a predetermined quantity of insulin such as, for example, a hypodermic syringe, an insulin pen, and an insulin pump. One such insulin pump is the Animas® 2020, a product which is manufactured by Animas Inc.

People with diabetes should maintain tight control over their lifestyle, so that they are not adversely affected by, for example, irregular food consumption or exercise. In addition, a physician dealing with a particular individual with diabetes may require detailed information on the individual's lifestyle to provide effective treatment or modification of treatment for controlling diabetes. Currently, one of the ways of monitoring the lifestyle of an individual with diabetes has been for the individual to keep a paper logbook of their lifestyle. Another way is for an individual to simply rely on remembering facts about their lifestyle and then relay these details to their physician on each visit.

The aforementioned methods of recording lifestyle information are inherently difficult, time consuming, and possibly inaccurate. Paper logbooks are not necessarily always carried by an individual and may not be accurately completed when required. Such paper logbooks are small and it is therefore difficult to enter detailed information requiring detailed descriptors of lifestyle events. Furthermore, an individual may often forget key facts about their lifestyle when questioned by a physician who has to manually review and interpret information from a hand-written notebook. There is no analysis provided by the paper logbook to distill or separate the component information. Also, there are no graphical reductions or summary of the information. Entry of data into a secondary data storage system, such as a database or other electronic system, requires a laborious transcription of information, including lifestyle data, into this secondary data storage. Difficulty of data recordation encourages retrospective entry of pertinent information that results in inaccurate and incomplete records.

There currently exist a number of portable electronic devices that can measure glucose levels in an individual and store the levels for recalling or uploading to another computer for analysis. One such device is the Accu-Check™ Complete™ System from Roche Diagnostics, which provides limited functionality for storing lifestyle data. However, the Accu-Check™ Complete™ System only permits a limited selection of lifestyle variables to be stored in a meter. There is a no intelligent feedback from values previously entered into the meter and the user interface is unintuitive for an infrequent user of the meter.

WO 2009/137661 A1 relates to a method for measuring and managing an analyte (e.g., blood glucose) in a bodily fluid including storing a therapeutic administration protocol in a memory module of an analyte measurement and management device and measuring the analyte in the bodily fluid sample using an analyte measurement module of the device. The method also includes calculating, with a processor module of the device, a recommended therapeutic agent dosage (for example, an insulin dosage) and a recommended administration time for user-activated delivery of the dosage by employing the therapeutic administration protocol. The method further includes displaying the recommended therapeutic agent dosage and administration time to a user on a visual display of the device, delivering a therapeutic agent dosage to the user via a user-activated therapeutic agent delivery device, and detecting the user-activated administration of the therapeutic agent using a delivery device communication module of the device. In addition, the method includes communicating the aforementioned detection to the processor module and/or memory module using the delivery device communication module.

## Summary of the Disclosure

In one example, a method of monitoring therapeutic bolus compliance with an analyte testing device and a therapeutic dispensing device is provided. Each of both devices includes a microprocessor coupled to a memory. The method may be achieved by: obtaining from the memory of the analyte testing device data including a number of hyperglycemic occurrences and a time stamp for each of the hyperglycemic occurrences; collecting from the memory of the therapeutic dispensing device a time stamp for each bolus of dispensed therapeutic; identifying a hyperglycemic occurrence that does not have at least one bolus of dispensed therapeutic within a predetermined time range of the hypoglycemic occurrence; calculating a percentage of hyperglycemic occurrences that do not have at least one bolus of dispensed therapeutic within the predetermined time range; and annunciating the percentage of hyperglycemic occurrences that do not have at least one bolus of dispensed therapeutic within the predetermined time range.

In yet another example, a method of monitoring insulin bolus compliance with an analyte testing device and a therapeutic dispensing device is provided. Each of both devices includes a microprocessor coupled to a memory. The method may be achieved by: obtaining from the memory of the analyte testing device data including a number of hyperglycemic occurrences and a time stamp for each of the hyperglycemic occurrences; collecting from the memory of the therapeutic dispensing device a time stamp for each bolus of dispensed therapeutic; identifying a hyperglycemic occurrence that has at least one bolus of dispensed therapeutic within a predetermined time range of the hypoglycemic occurrence; calculating a percentage of hyperglycemic occurrences that have at least one bolus of dispensed therapeutic within the predetermined time range; and annunciating the percentage of hyperglycemic occurrences that have at least one bolus of dispensed therapeutic within the predetermined time range.

In yet a further example, a diabetes management system is provided that includes a plurality of glucose test strips, test strip port connector, data management device. Each test strip is configured to receive a physiological sample. The test strip port connector is configured to receive the plurality of test strips. The data management device includes a housing, microprocessor, memory, display and power supply. The microprocessor is coupled to a memory, display, and power supply. The microprocessor is coupled to the test strip sensor to obtain data including a number of hyperglycemic occurrences and a time stamp for each of the hyperglycemic occurrences. The microprocessor is configured to collect a time stamp for each bolus of dispensed therapeutic so that a percentage of hyperglycemic occurrences that do not have at least one bolus of dispensed therapeutic within the predetermined time range is determined by the microprocessor.

In yet another example, a method of monitoring analyte-testing compliance with an analyte testing device and a therapeutic dispensing device is provided. Each of both devices includes a microprocessor coupled to a memory. The method may be achieved by: obtaining from the memory of the analyte testing device a time stamp for each of the glucose measurements; collecting from the memory of the therapeutic dispensing device data including a number of bolus events and a time stamp for each bolus event; identifying a bolus event that does not have at least one glucose measurement within a predetermined time range of the bolus event; calculating a percentage of bolus events that do not have at least one glucose measurement within the predetermined time range; and annunciating the percentage of bolus events that do not have at least one glucose measurement within the predetermined time range.

In a further example, a method of monitoring insulin bolus compliance with an analyte testing device and a therapeutic dispensing device is provided. Each of both devices includes a microprocessor coupled to a memory. The method may be achieved by: obtaining from the memory of the analyte testing device data including a number of glucose measurements and a time stamp for each of the glucose measurements; collecting from the memory of the therapeutic dispensing device data including a number of bolus events and a time stamp for each bolus event; identifying a bolus event that has at least one glucose measurement within a predetermined time range of the bolus event; calculating a percentage of bolus events that do have at least one glucose measurement within the predetermined time range; and annunciating a percentage of bolus events that do have at least one glucose measurement within the predetermined time range.

In another further example, a diabetes management system is provided that includes a plurality of glucose test strips, test strip port, and data management unit. Each of the test strips is configured to receive a physiological sample. The test strip port connector is configured to receive the plurality of test strips. The data management device includes a housing, microprocessor, memory, display and power supply. The microprocessor is coupled to a memory, display, and power supply with the microprocessor being disposed proximate the housing. The microprocessor is also coupled to the test strip sensor to obtain a time stamp for each of the glucose measurements. The microprocessor is configured to collect data including a number of bolus events and a time stamp for each bolus event so that a percentage of bolus events that do not have at least one glucose measurement within the predetermined time range are determined by the microprocessor.

In an example, a method of monitoring an average number of days in between cannula fills of a therapeutic dispensing device that delivers a therapeutic agent to a user via a cannula is provided. The method may be achieved by: (i) selecting a user interface option to perform a cannula fill procedure when a cannula is coupled to the therapeutic dispensing device; (ii) pumping an amount of therapeutic agent to fill the cannula; (iii) saving to a memory of the therapeutic dispensing device or an analyte- testing device a time stamp in which the cannula fill procedure was performed; (iv) repeating steps (i) to (iii) three or more times; (v) calculating an average number of days in between cannula fills; and (vi) annunciating the average number of days in between cannula fills.

According to the invention a method of monitoring bolus overrides with an analyte-testing device and a therapeutic dispensing device is provided as defined in claim 1.

These and other embodiments, features and advantages will become apparent to those skilled in the art when taken with reference to the following more detailed description of various exemplary embodiments of the invention in conjunction with the accompanying drawings that are first briefly described.

Brief Description of the Figures

The accompanying drawings, which are incorporated herein and constitute part of this specification, and, together with the general description given above and the detailed description given below, serve to explain features of the invention (wherein like numerals represent like elements).

Figure 1 illustrates a diabetes management system that includes an analyte measurement and management device and data communication devices.
Figure 2 illustrates a user interface of the analyte measurement and management device where data is transferred between a therapeutic dosing device and the analyte measurement and management device.
Figure 3A illustrates a top portion of a circuit board of the analyte measurement and management device.
Figure 3B illustrates a bottom portion of the circuit board of the analyte measurement and management device.
Figure 4 illustrates a schematic of the functional components of a therapeutic dosing device.
Figure 5 is a flow chart illustrating a method for performing a glucose test.
Figure 6 is a flow chart illustrating a method for setting up a bolus calculator.
Figure 7 is a flow chart illustrating a method for calculating an insulin bolus.
Figure 8 is a flow chart illustrating a method of monitoring therapeutic bolus compliance with the measurement and management device and the therapeutic dosing device.
Figure 9 is a flow chart illustrating a method of monitoring analyte-testing compliance with the measurement and management device and the therapeutic dosing device.
Figure 10 is a flow chart illustrating a method of monitoring an average number of days in between cannula fills.
Figure 11 illustrates an output on a report that provides a percentage of hyperglycemic results that have no boluses within +/- one hour, a percentage of boluses that have no glucose results within +/- one hour, and the average number of days in between cannula fills.
Figure 12 illustrates a cannula that is configured to be fluidically coupled to an insulin pump.
Figure 13 is a flow chart illustrating a method of indicating a bolus override that differs from a recommended bolus amount.
Figure 14 illustrates a screen-shot of a logbook that includes a bolus override, as indicated with either an up arrow or down arrow.

**Detailed Description of the Exemplary Figures**

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.
As used herein, the terms "about" or "approximately" for any numerical values or ranges indicate a suitable dimensional tolerance that allows the part or collection of components to function for its intended purpose as described herein. In addition, as used herein, the terms "patient," "host," "user," and "subject" refer to any human or animal subject and are not intended to limit the systems or methods to human use, although use of the subject invention in a human patient represents a preferred embodiment.

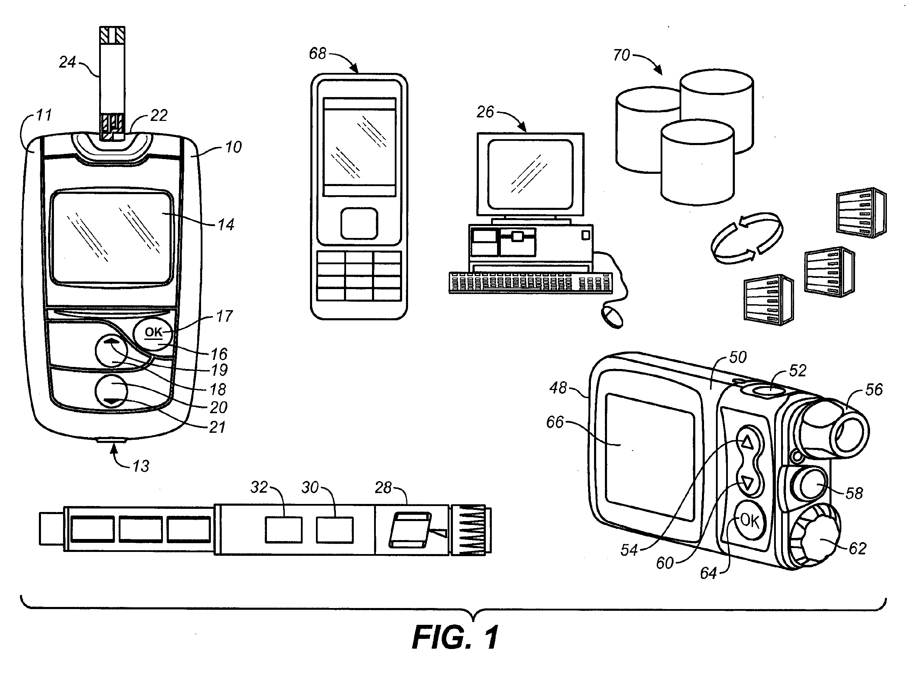

Figure 1 illustrates a diabetes management system that includes an analyte measurement and management device 10, therapeutic dosing devices (28 or 48), and data/communication devices (68, 26, or 70). Analyte measurement and management device 10 can be configured to wirelessly communicate with a handheld glucose-insulin data management unit or DMU such as, for example, an insulin pen 28, an insulin pump 48, a mobile phone 68, or through a combination of the exemplary handheld glucose-insulin data management unit devices in communication with a personal computer 26 or network server 70, as described herein. As used herein, the nomenclature "DMU" represents either individual unit 10, 28, 48, 68, separately or all of the handheld glucose-insulin data management units (28, 48, 68) usable together in a disease management system. Further, the analyte measurement and management device or DMU 10 is intended to include a glucose meter, a meter, an analyte measurement device, an insulin delivery device or a combination of or an analyte testing and drug delivery device. In an embodiment, analyte measurement and management device 10 may be connected to personal computer 26 with a cable. Alternatively, the device 10 may connect to the computer 26 or server via a suitable wireless technology.

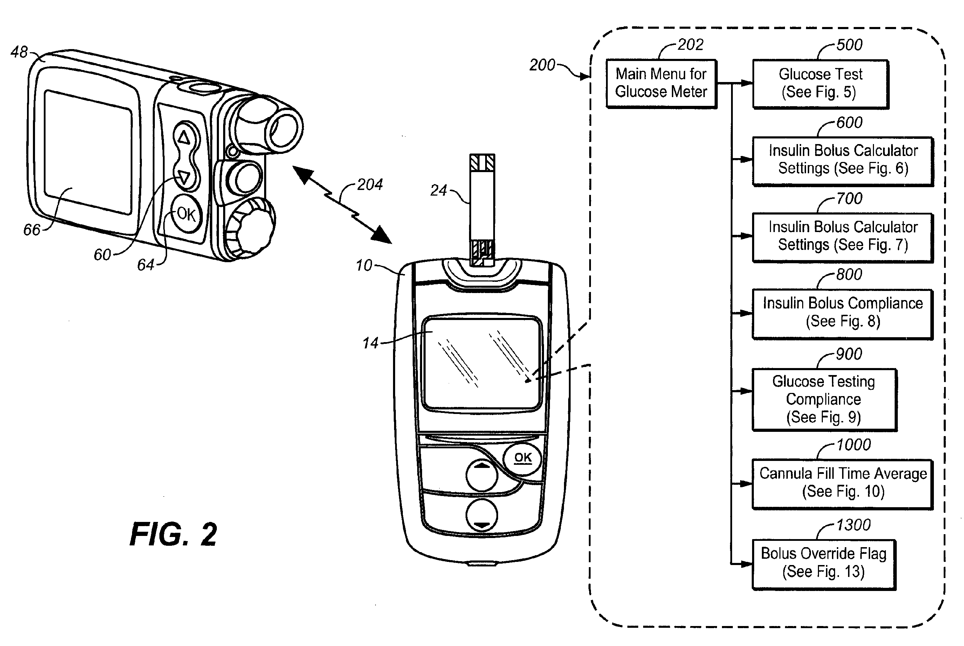

Figure 2 illustrates a user interface 200 of analyte measurement and management device 10. A microprocessor can be programmed to generally carry out the steps of user interface 200. The microprocessor can be part of a particular device, such as, for exampl,e, a glucose meter, an insulin pen, an insulin pump, a server, a mobile phone, personal computer, or mobile hand held device. Devices (10 and 48) can both be configured for bi-directional data transfer through a wireless signal 204. In an exemplary implementation, user interface 200 may provide recommendations, warnings, and compliance updates to a user as part of the user's diabetes management. In such embodiment, programs and methods for conducting user interface 200 may be stored on a non-volatile memory portion of glucose meter 10. Alternatively, interface 200 may be stored on a non-volatile memory portion of computer 26, cell phone 68, pump 48, or server 70. Steps and instructions of user interface 200 may be communicated on a communication output unit such as, for example, a display 14 of glucose meter 10 or the display 66 of the pump 48.

In such embodiment, the software for user interface 200 may be implemented using meter 10 and pump 48 without the need for an external computer, personal digital assistant, or mobile phone. However, in another embodiment, diabetes management 200 may be implemented using a personal computer where data is transferred bidirectionally from meter 10 and pump 48.

Note that recommendations, warnings, and compliance updates may be annunciated to a user. As used herein, the term "user" is intended to indicate primarily a mammalian subject (e.g., a person) who has diabetes but which term may also include a caretaker or a healthcare provider who is operating the meter 10 on behalf of the diabetes subject. As used here, the term "annunciated" and variations on the root term indicate that an announcement may be provided via text, audio, visual or a combination of all modes of communication to a user, a caretaker of the user, or a healthcare provider.

Glucose meter 10 can include a housing 11, user interface buttons (16, 18, and 20), a display 14, a strip port connector 22, and a data port 13, as illustrated in Figure 1. User interface buttons (16, 18, and 20) can be configured to allow the entry of data, navigation of menus, and execution of commands. Data can include values representative of analyte concentration, and/or information, which are related to the everyday lifestyle of an individual. Information, which is related to the everyday lifestyle, can include food intake, medication use, occurrence of health check-ups, and general health condition and exercise levels of an individual. Specifically, user interface buttons (16, 18, and 20) include a first user interface button 16, a second user interface button 18, and a third user interface button 20. User interface buttons (16, 18, and 20) include a first marking 17, a second marking 19, and a third marking 21, respectively, which allow a user to navigate through the user interface.

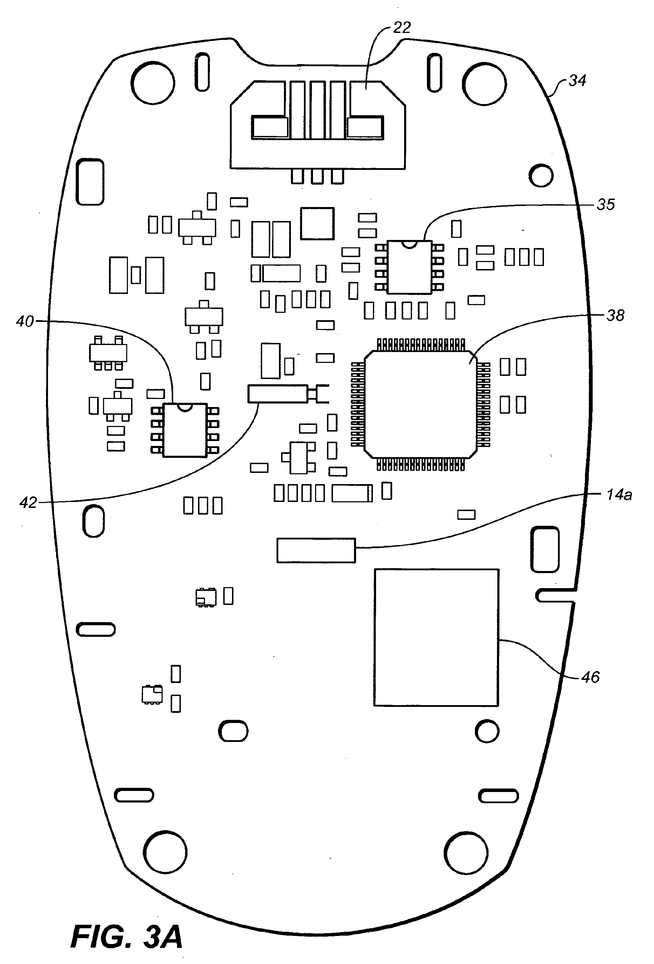

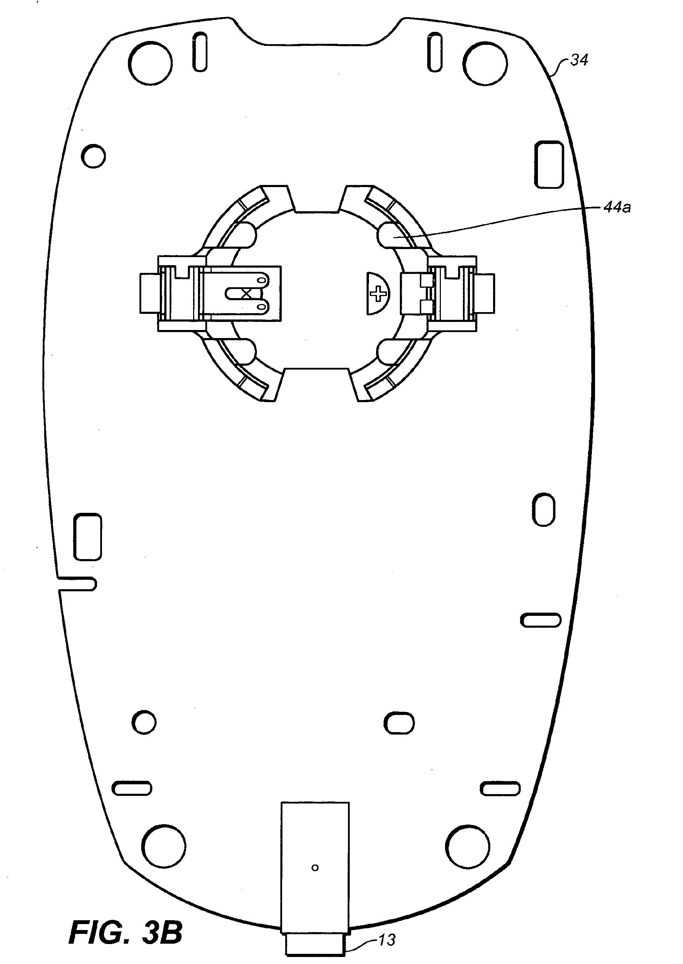

The electronic components of meter 10 can be disposed on a circuit board 34 that is within housing 11. Figures 3A and 3B illustrate the electronic components disposed on a top surface and a bottom surface of circuit board 34, respectively. On the top surface, the electronic components include a strip port connector 22, an operational amplifier circuit 35, a microcontroller 38, a display connector 14a, a non-volatile memory 40, a clock 42, and a first wireless module 46. On the bottom surface, the electronic components include a battery connector 44a and a data port 13. Microcontroller 38 can be electrically connected to strip port connector 22, operational amplifier circuit 35, first wireless module 46, display 14, non-volatile memory 40, clock 42, battery connector 44a, data port 13, and user interface buttons (16, 18, and 20).

Referring to Figure 3A, operational amplifier circuit 35 can include two or more operational amplifiers configured to provide a portion of the potentiostat function and the current measurement function. The potentiostat function can refer to the application of a test voltage between at least two electrodes of a test strip. The current function can refer to the measurement of a test current resulting from the applied test voltage. The current measurement may be performed with a current-to-voltage converter. Microcontroller 38 can be in the form of a mixed signal microprocessor (MSP) such as, for example, the Texas Instrument MSP 430. The MSP 430 can be configured to also perform a portion of the potentiostat function and the current measurement function. In addition, the MSP 430 can also include volatile and non-volatile memory. In another embodiment, many of the electronic components can be integrated with the microcontroller in the form of an application specific integrated circuit (ASIC).

Strip port connector 22 can be configured to form an electrical connection to the test strip. Display connector 14a can be configured to attach to display 14. Display 14 can be in the form of a liquid crystal display for reporting measured glucose levels, and for facilitating entry of lifestyle related information. Display 14 can optionally include a backlight. Data port 13 can accept a suitable connector attached to a connecting lead, thereby allowing glucose meter 10 to be linked to an external device such as a personal computer. Data port 13 can be any port that allows for transmission of data such as, for example, a serial, USB, or a parallel port. Clock 42 can be configured for measuring time and be in the form of an oscillating crystal. Battery connector 44a can be configured to be electrically connected to a power supply.

Test strip 24 can be in the form of an electrochemical glucose test strip, as illustrated in Figure 1. Test strip 24 can include one or more working electrodes and a counter electrode. Test strip 24 can also include a plurality of electrical contact pads, in which each electrode can be in electrical communication with at least one electrical contact pad. Strip port connector 22 can be configured to electrically interface to the electrical contact pads and form electrical communication with the electrodes. Test strip 24 can include a reagent layer that is disposed over at least one electrode. The reagent layer can include an enzyme and a mediator. Exemplary enzymes suitable for use in the reagent layer include glucose oxidase, glucose dehydrogenase (with pyrroloquinoline quinone co-factor, "PQQ"), and glucose dehydrogenase (with flavin adenine dinucleotide co-factor, "FAD"). An exemplary mediator suitable for use in the reagent layer includes ferricyanide, which in this case is in the oxidized form. The reagent layer can be configured to physically transform glucose into an enzymatic by-product and in the process generate an amount of reduced mediator (e.g., ferrocyanide) that is proportional to the glucose concentration. The working electrode can then measure a concentration of the reduced mediator in the form of a current. In turn, glucose meter 10 can convert the current magnitude into a glucose concentration.

Referring back to Figure 1, insulin pen 28 can include a housing, preferably elongated and of sufficient size to be handled by a human hand comfortably. The device 28 can be provided with an electronic module 30 to record dosage amounts delivered by the user. The device 28 may include a second wireless module 32 disposed in the housing that, automatically without prompting from a user, transmits a signal to first wireless module 46 of the DMU 10. The wireless signal can include, in an exemplary embodiment, data to (a) type of therapeutic agent delivered; (b) amount of therapeutic agent delivered to the user; or (c) time and date of therapeutic agent delivery.

A therapeutic delivery device can be in the form of a "user-activated" therapeutic delivery device, which requires a manual interaction between the device and a user (for example, by a user pushing a button on the device) to initiate a single therapeutic agent delivery event and that in the absence of such manual interaction deliver no therapeutic agent to the user. A non-limiting example of such a user-activated therapeutic agent delivery device is described in co-pending U.S. Non-Provisional Application No. 12/407173 (tentatively identified by Attorney Docket No. LFS-5180USNP); 12/417875 (tentatively identified by Attorney Docket No. LFS-5183USNP); and 12/540217 (tentatively identified by Attorney Docket No. DDI-5176USNP). Another non-limiting example of such a user-activated therapeutic agent delivery device is an insulin pen 28. Insulin pens can be loaded with a vial or cartridge of insulin, and can be attached to a disposable needle. Portions of the insulin pen can be reusable, or the insulin pen can be completely disposable. Insulin pens are commercially available from companies such as Novo Nordisk, Aventis, and Eli Lilly, and can be used with a variety of insulin, such as Novolog, Humalog, Levemir, and Lantus.

Referring to Figure 1, the therapeutic dosing device can also be a pump 48 that includes a housing 50, a backlight button 52, an up button 54, a cartridge cap 56, a bolus button 58, a down button 60, a battery cap 62, an OK button 64, and a display 66. Pump 48 can be configured to dispense medication such as, for example, insulin for regulating glucose levels.

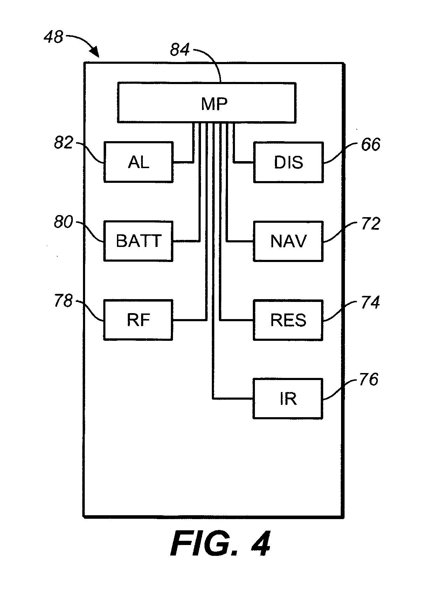

Referring to Figure 4, pump 48 includes the following functional components that are a display (DIS) 66, navigational buttons (NAV) 72, a reservoir (RES) 74, an infrared communication port (IR) 76, a radio frequency module (RF) 78, a battery (BAT) 80, an alarm module (AL) 82, and a microprocessor (MP) 84. Note that navigational buttons 72 can include up button 54, down button 60, and ok button 64.

People with diabetes will often have to perform several glucose tests and bolus several insulin doses to effectively manage the disease. The glucose concentration amount, time that the test was performed, and lifestyle data (e.g., meals and exercise) are factors often taken into account when calculating a proper insulin-dosing regimen. Applicant believes that users have difficulty determining the effectiveness and compliance with the insulin-dosing regimen. The following will describe a series of methods (500, 600, 700, 800, 900, 1000, and 1300) that a user may select within a main menu 202 for helping users better understand their compliance with managing their disease and guiding them to improving their compliance. As illustrated in Figure 2, the menu may include the following methods that are to perform a glucose test 500, provide an insulin bolus compliance update 600, a glucose testing compliance update 700, configure settings for insulin bolus calculator 800, calculate an insulin bolus 900, provide a cannula fill time average 1000, and output a bolus override flag 1300.

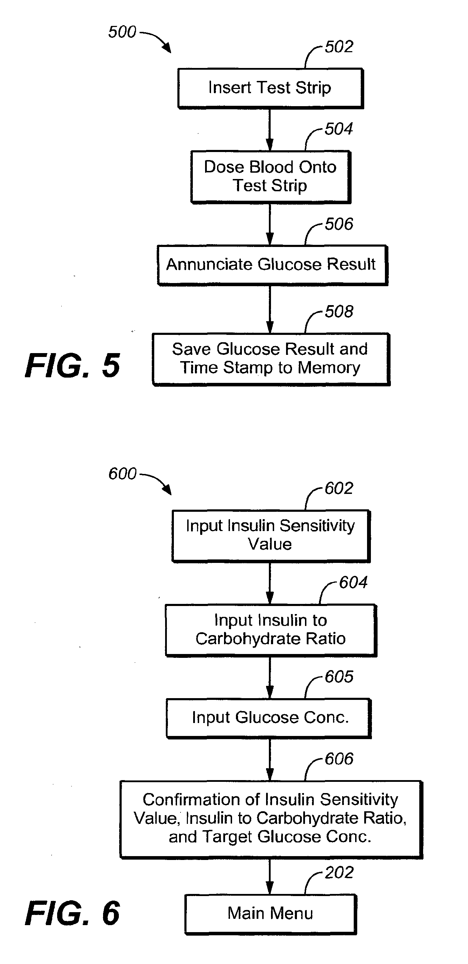

The glucose test 500 may include inserting an electrochemical test strip 24 into strip port connector 22 of glucose meter 10, as illustrated in a step 502 of Figure 5. A microprocessor can be programmed to generally carry out the steps of method 500. The microprocessor can be part of a particular device, such as, for example, a glucose meter, an insulin pen, an insulin pump, a server, a mobile phone, personal computer, or mobile hand held device. Once user interface prompts a user to dose blood, the user can dose a sample onto test strip 24, illustrated in a step 504. Glucose meter 10 can then perform the measurement and output a glucose result on display 14, as illustrated in a step 506. After the result is calculated, the glucose result and time stamp can be saved to a memory in the glucose meter 10, as illustrated in a step 508. The term time stamp can refer to the date and time that an event was performed, which in this case is a glucose measurement.

Figure 6 illustrates an embodiment 600 for configuring the set up of the bolus calculator 700. A microprocessor can be programmed to generally carry out the steps of method 600. The microprocessor can be part of a particular device, such as, for example, a glucose meter, an insulin pen, an insulin pump, a server, a mobile phone, personal computer, or mobile hand held device. A user may select an insulin sensitivity value, an insulin-to-carbohydrate ratio, and a target blood glucose value, as shown in steps 602, 604, and 605. More specifically, the user may select a discrete insulin sensitivity value and an insulin-to-carbohydrate ratio for a particular meal such as breakfast, lunch, or dinner. Insulin sensitivity values may range from about 5 mg/dL (or its conversion into mmol/L unit or millimole per liter) to about 300 mg/dL (or its conversion into mmol/L unit or millimole per liter). Insulin-to-carbohydrate ratio may range from about 5 grams to about 50 grams. Target blood glucose values may range from about 60 mg/dL (or its conversion into mmol/L unit or millimole per liter) to about 290 mg/dL (or its conversion into mmol/L unit or millimole per liter). Next, a confirmation of the insulin sensitivity value and an insulin-to-carbohydrate ratio may be annunciated to the user, as shown in a step 606, which is then followed by returning to main menu 202.

Briefly, three types of insulin boluses are described herein, which are an insulin bolus amount for: (a) carbohydrate coverage, (b) glucose correction, or (c) a combination thereof. The insulin bolus amount for carbohydrate coverage may be an amount of insulin needed to account for carbohydrates about to be consumed at a meal. The insulin bolus amount for a glucose measurement correction may be an amount of insulin needed to account for a user's measured glucose value that is greater than a targeted euglycemic glucose value. The combination (e.g., carbohydrate value and measured glucose value) correction may be an amount of insulin needed to account for carbohydrates about to be consumed and the user's measured glucose value.

Three types of insulin boluses are described herein, which are a Glucose Correction Dose, a Carbohydrate Coverage Dose, and a combination thereof. The Glucose Correction Dose is an amount of insulin needed to account for a user's recently measured glucose value that is greater than the euglycemic zone. The Carbohydrate Coverage Dose is an amount of insulin calculated based on the amount of carbohydrates to be consumed. The combination (e.g., carbohydrate value and measured glucose value) correction may be an amount of insulin needed to account for carbohydrates about to be consumed and the user's measured glucose value.

An embodiment of a Glucose Correction Dose is shown in Equation 1.

$$\text{Eq. 1} \quad \text{Glucose Correction Dose} = (\text{Current G} - \text{Target G}) \times \text{Insulin Sensitivity Factor}$$

The Glucose Correction Dose may be the amount of insulin needed to adjust the current measured glucose value or concentration to the euglycemic zone. The Current G and Target G may be the current measured glucose value or concentration and the target glucose value or concentration, respectively. The Insulin Sensitivity Factor may be a constant that is special to the user that relates to the proportional effectiveness of insulin.

The insulin bolus amount for carbohydrate coverage dose may be calculated by using Equation 2.

Eq. 2 Insulin bolus amount for carbohydrate coverage Dose = Carbohydrate Estimate x Insulin-to-Carbohydrate Ratio

The Carbohydrate Estimate may be the amount consumed by the user and the Insulin-to-Carbohydrate Ratio may be a constant that is special to the user relating to the proportional effectiveness of insulin on consumed carbohydrates. A total insulin dose may be calculated by summing together the Glucose Correction Dose and the Carbohydrate Anticipatory Dose.

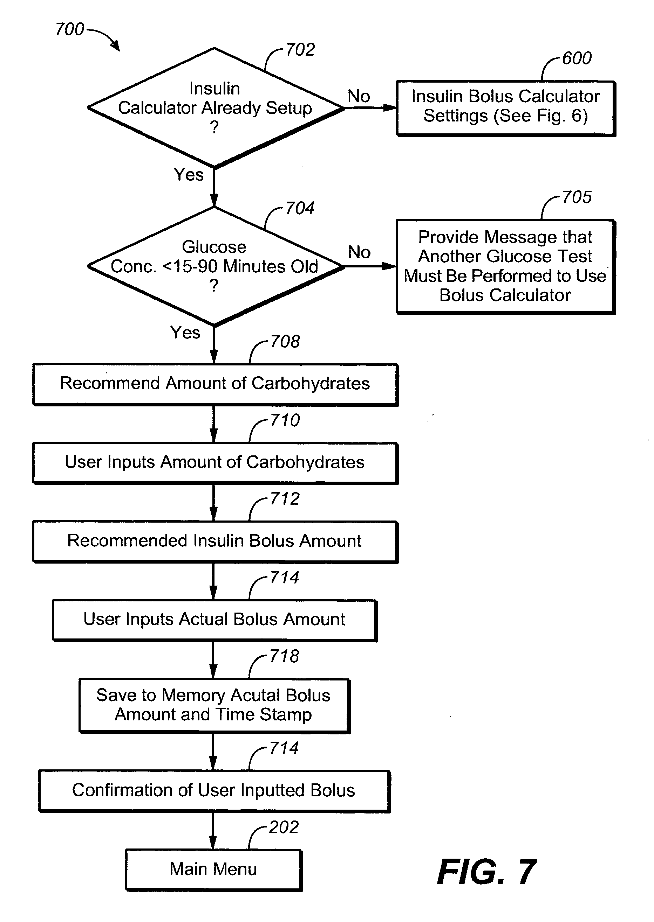

Figure 7 is a flow chart illustrating an embodiment of a method 700 for calculating an insulin bolus with a carbohydrate coverage and glucose correction. A microprocessor can be programmed to generally carry out the steps of method 700. The microprocessor can be part of a particular device, such as, for example, a glucose meter, an insulin pen, an insulin pump, a server, a mobile phone, personal computer, or mobile hand held device. Initially, the meter may determine whether the insulin calculator is already setup, as shown in a step 702. If the insulin calculator is not setup, then the method may move to an insulin bolus calculator settings function 600 (described above). If the insulin calculator has been setup, then the user interface 200 (which is implemented exemplarily in meter 10) may determine whether the last glucose value or concentration of the user measured is less than about 90 minutes to about 120 minutes old, as shown in a step 704. A message may be annunciated that another glucose test must be performed to use the bolus calculator, as shown in a step 705, when the last glucose value or concentration of the user measured is not less than about 90 minutes to about 120 minutes old.

A recommended amount of carbohydrates may be annunciated, as shown in a step 708, where the glucose value or concentration of the user is less than about 90 minutes to about 120 minutes old. The amount of carbohydrate may represent an amount that is about to be consumed by the user. The user has the option to input the recommended amount of carbohydrates or a different value, as shown in a step 710. As a non-limiting example, the amount of carbohydrates inputted may range from about zero to about 999 grams.

After inputting the amount of carbohydrates, a recommended insulin bolus may be annunciated, as shown in a step 712. Note that the recommended insulin bolus amount includes both an insulin bolus amount for carbohydrate coverage and an insulin correction of a recent measured glucose value of the user. The user has the option to input the recommended amount of insulin or a different value, as shown in a step 714, such as, for example, about zero to about 999 units. Next, the actual bolus amount and a time stamp may be saved to memory, as illustrated in a step 718. A confirmation of the inputted bolus amount may be annunciated to the user, as shown in a step 716, which is then followed by returning to main menu 202.

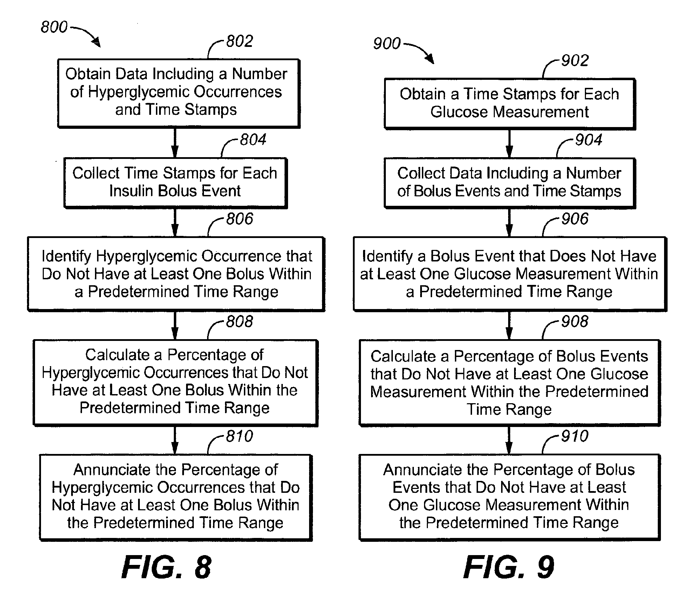

Figure 8 is a flow chart illustrating a method 800 of monitoring therapeutic bolus compliance with the measurement and management device 10 and the therapeutic dosing device 48. A microprocessor can be programmed to generally carry out the steps of method 800. The microprocessor can be part of a particular device, such as, for example, a glucose meter, an insulin pen, an insulin pump, a server, a mobile phone, personal computer, or mobile hand held device. As a general rule, a person with diabetes should take a bolus of insulin every time a hyperglycemic event is identified because clinical outcomes for individuals dramatically improve when a user reduces the duration of a hyperglycemic state. In an embodiment, a therapeutic bolus may represent an amount of insulin or an amount of drug for treating a disease such as, for example, diabetes. Typically, a bolus of therapeutic may be injected in its entirety into a user over a relatively short period of time such as, for example, about less than one minute.

Method 800 includes obtaining from the memory of the analyte testing device 10 data including a number of hyperglycemic occurrences and a time stamp for each of the hyperglycemic occurrences, as illustrated in a step 802. Before step 802, a user may assay a plurality of blood samples for glucose using a plurality of test strips 24 with glucose meter 10. In an embodiment, microcontroller 38 of glucose meter 10 may automatically identify glucose measurements that are hyperglycemic (e.g., glucose concentrations greater than about 200 milligrams per deciliter).

Method 800 also includes collecting from the memory of the therapeutic dispensing device 48 a time stamp for each bolus of dispensed therapeutic, as illustrated in a step 804. A user can initiate a bolus on pump 48 by manually inputting a bolus amount with navigational 72 buttons or by using method 700. The microprocessor 84 of pump 48 can record the date and time in which the bolus was dispensed and also the amount of insulin.

After steps 802 and 804, a microprocessor can identify a hyperglycemic occurrence that does not have at least one bolus of dispensed therapeutic within a predetermined time range of the hypoglycemic occurrence, as illustrated in a step 806. In an embodiment, the predetermined time range may be from about one hour before to about one hour after the time stamp of the hyperglycemic occurrence. For each hyperglycemic occurrence, the microprocessor may analyze all boluses to determine if at least one bolus has a time stamp that is within the predetermined time range. For example, if there is a hyperglycemic occurrence with a 2:00 pm time stamp, then the microprocessor can search for at least one bolus having a time stamp with a predetermined time range of +/- one hour, which in this case would be from about 1:00 pm to about 3:00 pm. If no bolus was found from about 1:00 pm to about 3:00, then the hyperglycemic occurrence would be identified as having no associated bolus within the predetermined time range of +/- one hour.

A percentage of hyperglycemic occurrences that do not have at least one bolus of dispensed therapeutic within the predetermined time range can be calculated, as illustrated in a step 808. The calculating can include determining the total number of hyperglycemic occurrences and the number of hyperglycemic occurrences that do not have at least one bolus of dispensed therapeutic within the predetermined time range. Next, the number of hyperglycemic occurrences that do not have at least one bolus of dispensed therapeutic within the predetermined time range can be divided by a total number of hyperglycemic occurrences. The calculation of step 808 can be calculated using Equation 3.

$$\text{Eq. 3} \quad \%H_{NB} = [H_{NB} / H_{T}] \times 100$$

The terms $\%H_{NB}$ is the percentage of hyperglycemic occurrences that do not have at least one bolus of dispensed therapeutic within the predetermined time range, $H_{NB}$ is the number of hyperglycemic occurrences that do not have at least one bolus of dispensed therapeutic within the predetermined time range, and $H_{T}$ is the total number of hyperglycemic occurrences.

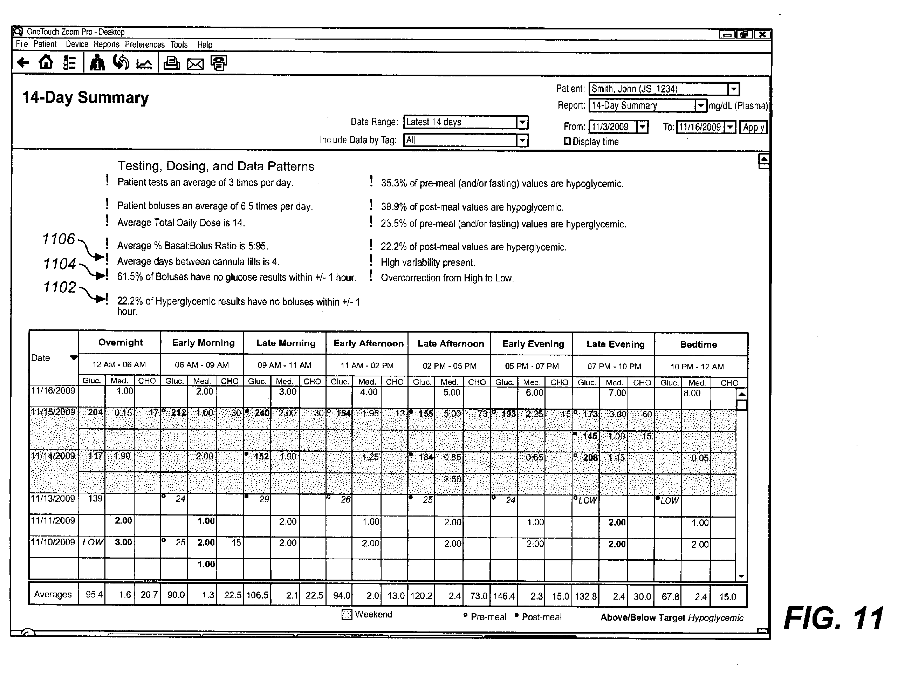

Once the percentage of step 808 has been calculated, the percentage of hyperglycemic occurrences that do not have at least one bolus of dispensed therapeutic within the predetermined time range can be annunciated, as illustrated in a step 810. Figure 11 illustrates an exemplary output on a portion 1102 of a report that provides a percentage of hyperglycemic results that have no boluses within +/- one hour. The output of step 810 may display on a screen of a personal computer configured into a special purpose health monitoring device, the analyte testing device, the therapeutic dispensing device, or other DMU (described above).

In an alternative embodiment to method 800, a percentage of hyperglycemic occurrences that "have" at least one bolus of dispensed therapeutic within a predetermined time range can be calculated instead of the percentage of hyperglycemic occurrences that "do not have" at least one bolus of dispensed therapeutic within a predetermined time range. The term $\%H_{WB}$ can be defined to be the percentage of hyperglycemic occurrences that "have" at least one bolus of dispensed therapeutic within a predetermined time range. The sum of the terms $\%H_{NB}$ and $\%H_{WB}$ will be about unity. In terms of providing an update to insulin bolus compliance, a user can employ either $\%H_{NB}$ or $\%H_{WB}$ for determining the adherence to recommended insulin therapy guidelines. In an embodiment, a user may have a target goal of a $\%H_{NB}$ of about zero or alternatively a $\%H_{WB}$ of about one.

Figure 9 is a flow chart illustrating a method 900 of monitoring analyte-testing compliance with the measurement and management device 10 and the therapeutic dosing device 48. A microprocessor can be programmed to generally carry out the steps of method 900. The microprocessor can be part of a particular device, such as, for example, a glucose meter, an insulin pen, an insulin pump, a server, a mobile phone, personal computer, or mobile hand held device. As a general rule, a person with diabetes should measure their glucose contemporaneous with an insulin bolus so that the amount of the bolus can be tailored to the amount of glucose in the person's blood. Implementing a more intelligent insulin-dosing regimen, based on current glucose measurements, that increases the duration of euglycemia, will dramatically improve clinical outcomes.

Method 900 includes obtaining from the memory of the analyte-testing device 10 a time stamp for each of the glucose measurements, as illustrated in a step 902. Before step 902, a user may assay a plurality of blood samples for glucose using a plurality of test strips 24 with glucose meter 10.

Method 900 also includes collecting from the memory of the therapeutic dispensing device 48 data including a number of bolus events and a time stamp for each bolus event, as illustrated in a step 904. A user can initiate a bolus on pump 48 by manually inputting a bolus amount with navigational 72 buttons or by using method 700. The microprocessor 84 of pump 48 can record the date and time in which the bolus was dispensed, the amount of insulin, and a total number of bolus events.

After steps 902 and 904, a microprocessor can identify a bolus event that does not have at least one glucose measurement within a predetermined time range of the bolus event, as illustrated in a step 906. In an embodiment, the predetermined time range may be from about one hour before to about one hour after the time stamp of the bolus event. For each bolus event, the microprocessor may analyze all glucose concentrations to determine if at least one glucose concentration has a time stamp that is within the predetermined time range. For example, if there is a bolus event with a 2:00 pm time stamp, then the microprocessor can search for at least one glucose measurement having a time stamp with a predetermined time range of +/- one hour, which in this case would be from about 1:00 pm to about 3:00 pm. Thus, if no glucose measurement was found from about 1:00 pm to about 3:00, then the bolus event would be identified as having no associated glucose measurement within the predetermined time range of +/- one hour.

A percentage of boluses that do not have at least one glucose measurement within the predetermined time range can be calculated, as illustrated in a step 908. The calculating can include determining the total number of bolus events and the number of bolus events that do not have at least one glucose measurement within the predetermined time range. Next, the number of bolus events that do not have at least one glucose measurement within the predetermined time range can be divided by a total number of bolus events. The calculation of step 908 can be calculated using Equation 4.

$$\text{Eq. 4} \quad \%B_{NG} = [B_{NG} / B_T] \times 100$$

The terms $\%B_{NG}$ is the percentage of bolus events that do not have at least one glucose measurement within the predetermined time range, $B_{NG}$ is the number of bolus events that do not have at least one glucose measurement within the predetermined time range, and $B_T$ is the total number of bolus events.

Once the percentage of step 908 has been calculated, the percentage of bolus events that do not have at least one glucose measurement within the predetermined time range can be annunciated, as illustrated in a step 910. Figure 11 illustrates an exemplary output on a portion 1104 of a report that provides a percentage of bolus events that have glucose measurements within +/- one hour. The output of step 910 may display on a screen of a personal computer, the analyte testing device, the therapeutic dispensing device, or other DMU (described above).

In an alternative embodiment to method 900, a percentage of bolus events that "have" at least one glucose measurement within a predetermined time range can be calculated instead of the percentage of bolus events that "do not have" at least one glucose measurements within a predetermined time range. The term $\%B_{WG}$ can be defined to be the percentage of bolus events that "have" at least one glucose measurement within a predetermined time range. As a result, the sum of the terms $\%B_{NG}$ and $\%B_{WG}$ will be about unity. In terms of providing an update to glucose testing compliance, a user can employ either $\%B_{NG}$ or $\%B_{WG}$ for determining the adherence to recommended insulin therapy guidelines. In an embodiment, a user may have a target goal of a $\%B_{NG}$ of about zero or alternatively a $\%B_{WG}$ of about one.

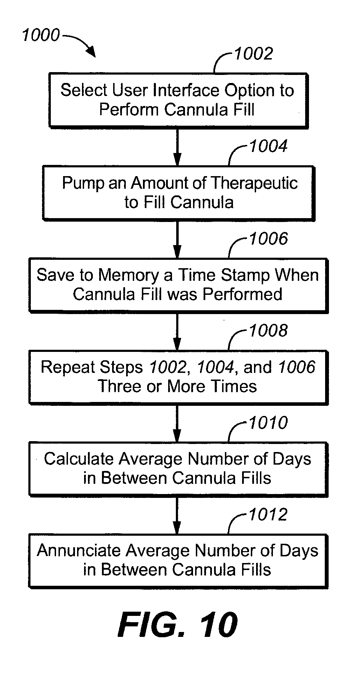

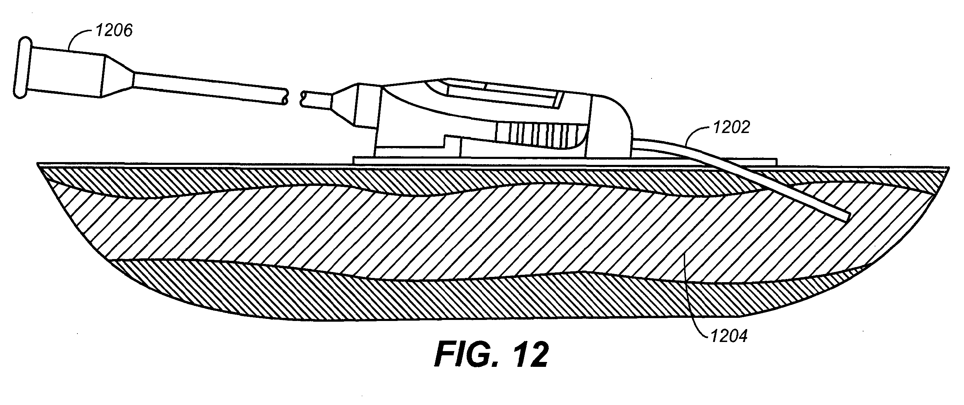

Figure 10 is a flow chart illustrating a method 1000 of monitoring an average number of days in between cannula fills. A microprocessor can be programmed to generally carry out the steps of method 1000. The microprocessor can be part of a particular device, such as, for example, a glucose meter, an insulin pen, an insulin pump, a server, a mobile phone, personal computer, or mobile hand held device. A cannula is a small tube that is fluidically connected to an insulin pump for dispensing drug into the body. Figure 12 illustrates a side view of a cannula 1202 that has been inserted into a subcutaneous layer 1204 of the skin. Cannula 1202 may be attached to a cannula housing 1204 that is fluidically connected to a luer fitting 1206. An insulin pump can then connect to luer fitting 1206. The cannula may remain in the body after being inserted with a needle that is subsequently removed. In some systems, the cannula should be changed with a particular frequency (every 3 days) to mitigate the risk of clogging. Additional details may be found regarding cannulas in U.S. Patent No.'s 7,052,483 and 6,572,586, which are hereby fully incorporated herein.

Method 1000 includes selecting a user interface option to perform a cannula fill procedure when a cannula is coupled to a therapeutic dispensing device, as illustrated in a step 1002. After the cannula fill option is selected, a user must physically attach a new cannula to the pump and if necessary remove the old cannula.

Once the new cannula is attached an amount of therapeutic is pumped so that the cannula is filled, as illustrated in a step 1004. In an embodiment, a user can select a user interface option to prime the cannula. Next, a time stamp in which the cannula fill procedure was performed can be saved to a memory, as illustrated in a step 1006. Note that the time stamp may be saved to a memory of the therapeutic dispensing device, the analyte testing device, a personal computer, or other DMU. The steps 1002, 1004, and 1006 can be repeated three or more times, as illustrated in a step 1008.

After step 1008, a microprocessor can calculate an average number of days in between cannula fills, as illustrated in a step 1010. The calculation of step 1010 can be calculated using Equation 5.

$$\text{Eq. 5} \quad C_{avg} = \frac{\sum_{i=1}^{N} C_i}{(N-1)}$$

The terms $C_{avg}$ is an average number of days between cannula fills, $C_i$ is the number of days between two consecutive cannula fills, and N is a total number of cannula fills.

Once the average number of days in between cannula fills for step 1010 has been calculated, it can be annunciated, as illustrated in a step 1012. The output of step 1012 may display on a screen of a personal computer, the analyte testing device, the therapeutic dispensing device, or other DMU (described above). In an embodiment, an alert can be annunciated to a user where the average number of days in between cannula fills is greater than about three days.

Under certain circumstances, a user may adjust a recommended bolus to be lower or higher due to factors not typically accounted for in a basic insulin calculator. For instance, the type of insulin (e.g., fast or slow acting) or type of carbohydrate (e.g., simple or complex) may cause a user to adjust the recommended bolus amount. However, if the adjusted bolus amount results in hypo or hyperglycemia, the user or HCP may want to review a logbook of adjusted insulin boluses to see whether a particular adjustment was higher or lower.

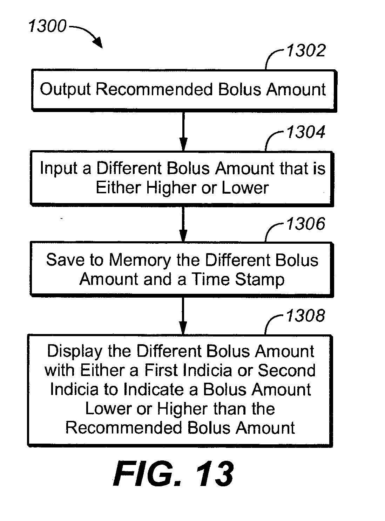

Figure 13 is a flow chart illustrating a method 1300 for indicating a bolus override that differs from a recommended bolus amount. A microprocessor can be programmed to generally carry out the steps of method 1300. The microprocessor can be part of a particular device, such as, for example, a glucose meter, an insulin pen, an insulin pump, a server, a mobile phone, personal computer, or mobile hand held device. When using an insulin bolus calculator, a recommended bolus amount can be displayed on a display of a special purpose computer 26, either an analyte testing device 10 or a therapeutic dispensing device 48, as shown in a step 1302. A user can either accept the recommended bolus amount, or alternatively can override the bolus amount by inputting a different bolus amount that is either higher or lower, as shown in a step 1304. Where the bolus amount is overridden, the different bolus amount and a time stamp can be saved to a memory of either analyte testing device 10 or therapeutic dispensing device 48, as shown in a step 1306. Next, the different bolus amount can be displayed with either a first indicia or second indicia to indicate that the adjusted bolus amount was lower or higher than the recommended amount, as shown in a step 1308.

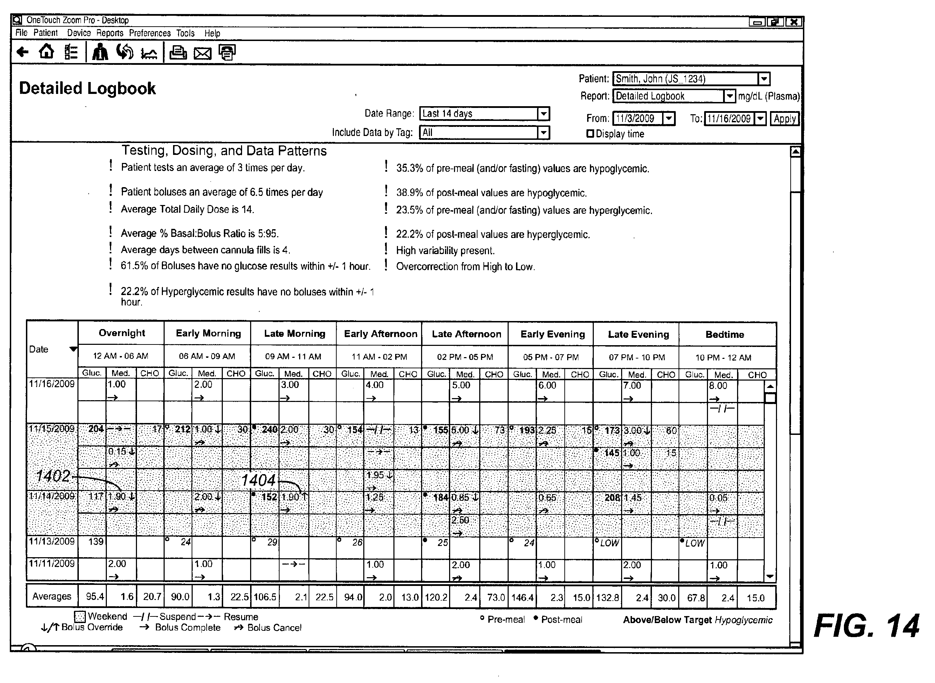

In an embodiment, the different bolus amount with either first or second indicia can be displayed on a display of a special purpose computer 26, analyte testing device 10 or therapeutic dispensing device 48. In another embodiment, the different bolus amount with either first or second indicia can displayed on a display of personal computer 26 after the data has been transferred. The first and second indicia may be represented by an up arrow or down arrow, as illustrated in sections 1404 and 1402 of the screen-shot of Figure 14. The first or the second indicia may be located immediately adjacent to the recommended bolus amount.

The different bolus amount can be located within a cell of a logbook. More specifically, different bolus amounts with either an up or down arrow are shown in the medication column (denoted as "Med") of Figure 14. A logbook can include a plurality of cells organized into a two dimensional array where a first dimension represents time intervals during one day and a second dimension represents time intervals of several days. For example, the time intervals of the first dimension can be represented in columns labeled as "Overnight," "Early Morning," "Late Morning," "Early Afternoon," "Late Afternoon," "Early Evening," "Late Evening," and "Bedtime." The time intervals of the second dimension can be represented as a plurality of rows in which each row is a single day.

It is noted that the various methods described herein can be used to generate software codes using off-the-shelf software development tools such as, for example, Visual Studio 6.0, Windows 2000 Server, and SQL Server 2000. The methods, however, may be transformed into other software languages depending on the requirements and the availability of new software languages for coding the methods. Additionally, the various methods described, once transformed into suitable software codes, may be embodied in any computer-readable storage medium that, when executed by a suitable microprocessor or computer, are operable to carry out the steps described in these methods along with any other necessary steps.

**Claims**

1. A method of monitoring bolus overrides with an analyte testing device and a therapeutic dispensing device, in which each of both devices includes a microprocessor coupled to a memory, the method comprising:

(i) annunciating a recommended bolus amount on a display of either the analyte testing device or a therapeutic dispensing device;
(ii) inputting a different bolus amount that is either higher or lower than the recommended bolus amount;
(iii) saving to a memory of the therapeutic dispensing device or an analyte testing device the different bolus amount and a time stamp; and
(iv) displaying the different bolus amount with either a first indicia indicating a bolus amount higher than the recommended bolus amount or a second indicia indicating a bolus amount lower than the recommended bolus amount, **characterised in that**

the different bolus amount is located within a cell of a logbook displayed via a personal computer, the logbook comprising a plurality of cells organized into a two dimensional array where a first dimension represents time intervals during one day and a second dimension represents time intervals of several days.

2. The method of Claim 1, in which the first or the second indicia is immediately adjacent to the recommended bolus amount.

3. The method of Claim 1, in which the first indicia comprises an up arrow and the second indicia comprises a down arrow.

4. The method of Claim 1 further comprising:

before the annunciating, assaying a blood sample to measure a glucose concentration; and
calculating the recommended bolus amount based on the measured glucose concentration, an insulin sensitivity value, and a target glucose concentration.

5. The method of Claim 1 further comprising:

before the annunciating, inputting an amount of carbohydrate into the analyte testing device or the therapeutic dispensing device; and
calculating the recommended bolus amount based on the measured glucose concentration, an insulin to carbohydrate ratio, and a target glucose concentration.

**Patentansprüche**

1. Verfahren zum Überwachen von Bolusbeeinflussungen mit einer Analyt-Testvorrichtung und einer therapeutischen Abgabevorrichtung, wobei jede der beiden Vorrichtungen einen Mikroprozessor enthält, der mit einem Speicher gekoppelt ist, wobei das Verfahren Folgendes umfasst:

(i) Ankündigen einer empfohlenen Bolusmenge auf einer Anzeige entweder der Analyt-Testvorrichtung oder einer therapeutischen Abgabevorrichtung;
(ii) Eingeben einer anderen Bolusmenge, die entweder höher oder niedriger als die empfohlene Bolusmenge ist;
(iii) Speichern der anderen Bolusmenge und eines Zeitstempels in einem Speicher der therapeutischen Abgabevorrichtung oder einer Analyt-Testvorrichtung und
(iv) Anzeigen der anderen Bolusmenge entweder mit einem ersten Index, der eine Bolusmenge angibt, die höher als die empfohlene Bolusmenge ist, oder einem zweiten Index, der eine Bolusmenge angibt, die niedriger als die empfohlene Bolusmenge ist,

**dadurch gekennzeichnet, dass**
die andere Bolusmenge innerhalb einer Zelle eines Logbuchs angeordnet ist, das durch einen Personalcomputer angezeigt wird, wobei das Logbuch mehrere Zellen umfasst, die in einem zweidimensionalen Feld eingeordnet sind, in dem eine erste Dimension Zeitintervalle während eines Tages darstellt und eine zweite Dimension Zeitintervalle von mehreren Tagen darstellt.

**2.** Verfahren nach Anspruch 1, bei dem der erste oder der zweite Index unmittelbar neben der empfohlenen Bolusmenge steht.

**3.** Verfahren nach Anspruch 1, bei dem der erste Index einen Pfeil nach oben umfasst und der zweite Index einen Pfeil nach unten umfasst.

**4.** Verfahren nach Anspruch 1, das ferner Folgendes umfasst:

vor dem Ankündigen das Untersuchen einer Blutprobe, um eine Glukosekonzentration zu messen, und
das Berechnen der empfohlenen Bolusmenge anhand der gemessenen Glukosekonzentration, eines Insulinempfindlichkeitswerts und einer Zielglukosekonzentration.

**5.** Verfahren nach Anspruch 1, das ferner Folgendes umfasst:

vor dem Ankündigen Eingeben einer Menge von Kohlenhydraten in die Analyt-Testvorrichtung oder die therapeutische Abgabevorrichtung und
Berechnen der empfohlenen Bolusmenge anhand der gemessenen Glukosekonzentration, eines Verhältnisses von Insulin zu Kohlenhydraten und einer Zielglukosekonzentration.

**Revendications**

**1.** Procédé pour surveiller des dépassements de bolus avec un dispositif de test d'analyte et un dispositif de distribution thérapeutique, où chacun des deux dispositifs comprend un microprocesseur couplé à une mémoire, le procédé comprenant les étapes suivantes :

(i) indiquer une quantité de bolus recommandée sur un écran soit du dispositif de test d'analyte, soit du dispositif de distribution thérapeutique ;
(ii) entrer une quantité de bolus différente qui est soit supérieure, soit inférieure à la quantité de bolus recommandée ;
(iii) sauvegarder dans une mémoire du dispositif de distribution thérapeutique ou du dispositif de test d'analyte la quantité différente de bolus et une estampille temporelle ; et
(iv) afficher la quantité de bolus différente avec soit un premier indice indiquant une quantité de bolus supérieure à la quantité de bolus recommandée, soit un second indice indiquant une quantité de bolus inférieure à la quantité de bolus recommandée, **caractérisé en ce que** :

la quantité de bolus différente est située dans une cellule d'un journal de bord affiché via un ordinateur personnel, le journal de bord comprenant une pluralité de cellules organisées dans un tableau à deux dimensions où une première dimension représente des intervalles de temps au cours d'une journée et une seconde dimension représente des intervalles de temps sur plusieurs jours.

**2.** Procédé selon la revendication 1, dans lequel le premier ou le second indice est immédiatement adjacent à la quantité de bolus recommandée.

**3.** Procédé selon la revendication 1, dans lequel le premier indice comprend une flèche vers le haut, et le second indice comprend une flèche vers le bas.

**4.** Procédé selon la revendication 1, comprenant en outre les étapes suivantes :

avant l'indication, analyser un échantillon de sang pour mesurer une concentration de glucose ; et
calculer la quantité de bolus recommandée sur la base de la concentration de glucose mesurée, d'une valeur de sensibilité à l'insuline, et d'une concentration de glucose cible.

**5.** Procédé selon la revendication 1, comprenant en outre les étapes suivantes :

avant l'indication, entrer une quantité d'hydrate de carbone dans le dispositif de test d'analyte ou dans le dispositif de distribution thérapeutique ; et

calculer la quantité de bolus recommandée sur la base de la concentration de glucose mesurée, d'un rapport d'insuline sur hydrate de carbone, et d'une concentration de glucose cible.

FIG. 1

200

202 Main Menu for Glucose Meter

500 Glucose Test (See Fig. 5)

600 Insulin Bolus Calculator Settings (See Fig. 6)

700 Insulin Bolus Calculator Settings (See Fig. 7)

800 Insulin Bolus Compliance (See Fig. 8)

900 Glucose Testing Compliance (See Fig. 9)

1000 Cannula Fill Time Average (See Fig. 10)

1300 Bolus Override Flag (See Fig. 13)

204

24

10

14

OK

48

60

64

66

*FIG. 2*

**FIG. 3A**

FIG. 3B

**FIG. 4**

500

502

Insert Test Strip

504

Dose Blood Onto
Test Strip

506

Annunciate Glucose Result

508

**FIG. 5**

Save Glucose Result and
Time Stamp to Memory

600

602

Input Insulin Sensitivity
Value

604

Input Insulin to
Carbohydrate Ratio

605

Input Glucose Conc.

606

Confirmation of Insulin Sensitivity
Value, Insulin to Carbohydrate Ratio,
and Target Glucose Conc.

202

**FIG. 6**

Main Menu

700

702

Insulin
Calculator Already Setup
?

No → Insulin Bolus Calculator
Settings (See Fig. 6)

600

Yes ↓

704

Glucose
Conc. <15-90 Minutes Old
?

No → Provide Message that
Another Glucose Test
Must Be Performed to Use
Bolus Calculator

705

Yes ↓

708

Recommend Amount of Carbohydrates

↓ 710

User Inputs Amount of Carbohydrates

↓ 712

Recommended Insulin Bolus Amount

↓ 714

User Inputs Actual Bolus Amount

↓ 718

Save to Memory Acutal Bolus
Amount and Time Stamp

↓ 714

Confirmation of User Inputted Bolus

↓ 202

Main Menu

**FIG. 7**

800 ⟍

802

Obtain Data Including a Number
of Hyperglycemic Occurrences
and Time Stamps

↓ 804

Collect Time Stamps for Each
Insulin Bolus Event

↓ 806

Identify Hyperglycemic Occurrence that
Do Not Have at Least One Bolus Within
a Predetermined Time Range

↓ 808

Calculate a Percentage of
Hyperglycemic Occurrences that Do Not
Have at Least One Bolus Within the
Predetermined Time Range

↓ 810

Annunciate the Percentage of
Hyperglycemic Occurrences that Do
Not Have at Least One Bolus Within
the Predetermined Time Range

**FIG. 8**

900 ⟍

902

Obtain a Time Stamps for Each
Glucose Measurement

↓ 904

Collect Data Including a Number
of Bolus Events and Time Stamps

↓ 906

Identify a Bolus Event that Does Not Have
at Least One Glucose Measurement Within
a Predetermined Time Range

↓ 908

Calculate a Percentage of Bolus Events
that Do Not Have at Least One Glucose
Measurement Within the Predetermined
Time Range

↓ 910

Annunciate the Percentage of Bolus
Events that Do Not Have at Least
One Glucose Measurement Within
the Predetermined Time Range

**FIG. 9**

1000 ⟍

```
                                        ┌─1002
        ┌──────────────────────────────┐
        │  Select User Interface Option to │
        │     Perform Cannula Fill       │
        └──────────────────────────────┘
                        │
                        ▼        ┌─1004
        ┌──────────────────────────────┐
        │   Pump an Amount of Therapeutic │
        │        to Fill Cannula         │
        └──────────────────────────────┘
                        │
                        ▼        ┌─1006
        ┌──────────────────────────────┐
        │  Save to Memory a Time Stamp When │
        │     Cannula Fill was Performed  │
        └──────────────────────────────┘
                        │
                        ▼        ┌─1008
        ┌──────────────────────────────┐
        │  Repeat Steps 1002, 1004, and 1006 │
        │        Three or More Times     │
        └──────────────────────────────┘
                        │
                        ▼        ┌─1010
        ┌──────────────────────────────┐
        │  Calculate Average Number of Days │
        │      in Between Cannula Fills   │
        └──────────────────────────────┘
                        │
                        ▼        ┌─1012
        ┌──────────────────────────────┐
        │  Annunciate Average Number of Days │
        │      in Between Cannula Fills   │
        └──────────────────────────────┘
```

# FIG. 10

**OneTouch Zoom Pro - Desktop**

File  Patient  Device  Reports  Preferences  Tools  Help

## 14-Day Summary

Patient: Smith, John (JS_1234) ▼
Report: 14-Day Summary ▼ mg/dL (Plasma)

Date Range: Latest 14 days ▼
From: 11/3/2009 ▼  To: 11/16/2009 ▼  Apply
Include Data by Tag: All ▼
☐ Display time

### Testing, Dosing, and Data Patterns

! Patient tests an average of 3 times per day.

! Patient boluses an average of 6.5 times per day.

! Average Total Daily Dose is 14.

1106 ! Average % Basal:Bolus Ratio is 5:95.

1104 ! Average days between cannula fills is 4.

! 61.5% of Boluses have no glucose results within +/- 1 hour.

1102 ! 22.2% of Hyperglycemic results have no boluses within +/- 1 hour.

! 35.3% of pre-meal (and/or fasting) values are hypoglycemic.

! 38.9% of post-meal values are hypoglycemic.

! 23.5% of pre-meal (and/or fasting) values are hyperglycemic.

! 22.2% of post-meal values are hyperglycemic.

! High variability present.

! Overcorrection from High to Low.

| Date ▼ | Overnight 12 AM - 06 AM | | | Early Morning 06 AM - 09 AM | | | Late Morning 09 AM - 11 AM | | | Early Afternoon 11 AM - 02 PM | | | Late Afternoon 02 PM - 05 PM | | | Early Evening 05 PM - 07 PM | | | Late Evening 07 PM - 10 PM | | | Bedtime 10 PM - 12 AM | | |
|---|---|---|---|---|---|---|---|---|---|---|---|---|---|---|---|---|---|---|---|---|---|---|---|---|
| | Gluc. | Med. | CHO | Gluc. | Med. | CHO | Gluc. | Med. | CHO | Gluc. | Med. | CHO | Gluc. | Med. | CHO | Gluc. | Med. | CHO | Gluc. | Med. | CHO | Gluc. | Med. | CHO |
| 11/16/2009 | | 1.00 | | | 2.00 | | | 3.00 | | | 4.00 | | | 5.00 | | | 6.00 | | | 7.00 | | | 8.00 | |
| 11/15/2009 | 204 | 0.15 | 17 | 212 | 1.00 | 30 | 240 | 2.00 | 30 | 154 | 1.95 | 13 | 155 | 5.00 | 73 | 193 | 2.25 | 15 | 173 | 3.00 | 60 | | | |
| | | | | | | | | | | | | | | | | | | | 145 | 1.00 | 15 | | | |
| 11/14/2009 | 117 | 1.90 | | | 2.00 | | 152 | 1.90 | | | 1.25 | | 184 | 0.85 | | | 0.65 | | 208 | 1.45 | | | 0.05 | |
| | | | | | | | | | | | | | | 2.50 | | | | | | | | | | |
| 11/13/2009 | 139 | | | 24 | | | 29 | | | 26 | | | 25 | | | 24 | | | LOW | | | LOW | | |
| 11/11/2009 | | 2.00 | | | 1.00 | | | 2.00 | | | 1.00 | | | 2.00 | | | 1.00 | | | 2.00 | | | 1.00 | |
| 11/10/2009 | LOW | 3.00 | | 25 | 2.00 | 15 | | 2.00 | | | 2.00 | | | 2.00 | | | 2.00 | | | 2.00 | | | 2.00 | |
| | | | | | 1.00 | | | | | | | | | | | | | | | | | | | |
| Averages | 95.4 | 1.6 | 20.7 | 90.0 | 1.3 | 22.5 | 106.5 | 2.1 | 22.5 | 94.0 | 2.0 | 13.0 | 120.2 | 2.4 | 73.0 | 146.4 | 2.3 | 15.0 | 132.8 | 2.4 | 30.0 | 67.8 | 2.4 | 15.0 |

☐ Weekend     ° Pre-meal   • Post-meal     **Above/Below Target** *Hypoglycemic*

## FIG. 11

FIG. 12

*1300*

*1302*

Output Recommended Bolus Amount

*1304*

Input a Different Bolus Amount that is Either Higher or Lower

*1306*

Save to Memory the Different Bolus Amount and a Time Stamp

*1308*

Display the Different Bolus Amount with Either a First Indicia or Second Indicia to Indicate a Bolus Amount Lower or Higher than the Recommended Bolus Amount

## FIG. 13

OneTouch Zoom Pro - Desktop

File  Patient  Device  Reports  Preferences  Tools  Help

# Detailed Logbook

Patient: Smith, John (JS_1234) ▾
Report: Detailed Logbook ▾ mg/dL (Plasma)

Date Range: Last 14 days ▾

Include Data by Tag: All ▾

From: 11/3/2009 ▾  To: 11/16/2009 ▾ Apply
☐ Display time

## Testing, Dosing, and Data Patterns

! Patient tests an average of 3 times per day.

! Patient boluses an average of 6.5 times per day

! Average Total Daily Dose is 14.

! Average % Basal:Bolus Ratio is 5:95.

! Average days between cannula fills is 4.

! 61.5% of Boluses have no glucose results within +/- 1 hour.

! 22.2% of Hyperglycemic results have no boluses within +/- 1 hour.

! 35.3% of pre-meal (and/or fasting) values are hypoglycemic.

! 38.9% of post-meal values are hypoglycemic.

! 23.5% of pre-meal (and/or fasting) values are hyperglycemic.

! 22.2% of post-meal values are hyperglycemic.

! High variability present.

! Overcorrection from High to Low.

| Date ▾ | Overnight 12 AM - 06 AM | | | Early Morning 06 AM - 09 AM | | | Late Morning 09 AM - 11 AM | | | Early Afternoon 11 AM - 02 PM | | | Late Afternoon 02 PM - 05 PM | | | Early Evening 05 PM - 07 PM | | | Late Evening 07 PM - 10 PM | | | Bedtime 10 PM - 12 AM | | |
|---|---|---|---|---|---|---|---|---|---|---|---|---|---|---|---|---|---|---|---|---|---|---|---|---|
| | Gluc. | Med. | CHO | Gluc. | Med. | CHO | Gluc. | Med. | CHO | Gluc. | Med. | CHO | Gluc. | Med. | CHO | Gluc. | Med. | CHO | Gluc. | Med. | CHO | Gluc. | Med. | CHO |
| 11/16/2009 | | 1.00 → | | | 2.00 → | | | 3.00 → | | | 4.00 → | | | 5.00 → | | | 6.00 → | | | 7.00 → | | | 8.00 → —/ /— | |
| 11/15/2009 | 204 | —→— | 17 | 212 | 1.00 ↓ ↗ | 30 | 240 | 2.00 → | 30 | 154 | —/ /— | 13 | 155 | 5.00 ↓ ↗ | 73 | 193 | 2.25 ↗ | 15 | 173 | 3.00 ↓ ↗ | 60 | | | |
| | | 0.15 ↓ ↗ | | | | | | | | | | | | —→— | | | | | 145 | 1.00 → | 15 | | | |
| | | | | | | | | | | | 1.95 ↓ → | | | | | | | | | | | | | |
| 11/14/2009 | 117 | 1.90 ↓ ↗ | | | 2.00 ↓ ↗ | | 152 | 1.90 ↑ | | | 1.25 → | | 184 | 0.85 ↓ ↗ | | | 0.65 ↗ | | 208 | 1.45 → | | | 0.05 → | |
| | | | | | | | | | | | | | | 2.50 → | | | | | | | | | —/ /— | |
| 11/13/2009 | 139 | | | 24 | | | 29 | | | 26 | | | 25 | | | 24 | | | LOW | | | LOW | | |
| 11/11/2009 | | 2.00 → | | | 1.00 → | | | —→— | | | 1.00 → | | | 2.00 → | | | 1.00 → | | | 2.00 → | | | 1.00 → | |
| Averages | 95.4 | 1.6 | 20.7 | 90.0 | 1.3 | 22.5 | 106.5 | 2.1 | 22.5 | 94.0 | 2.0 | 13.0 | 120.2 | 2.4 | 73.0 | 146.4 | 2.3 | 15.0 | 132.8 | 2.4 | 30.0 | 67.8 | 2.4 | 15.0 |

1402
1404

▨ Weekend  —/ /— Suspend —→— Resume
↓/↑ Bolus Override  → Bolus Complete  ↗ Bolus Cancel

° Pre-meal  • Post-meal  **Above/Below Target** *Hypoglycemic*

# FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009137661 A1
- US 407173 A
- US 12417875 B
- US 12540217 B
- US 7052483 B
- US 6572586 B